# EUROPEAN PATENT APPLICATION

(11) **EP 0 860 505 A1**
(43) Date of publication of application: **26.08.1998**
(21) Application number: 96935435.6
(22) Date of filing: 24.10.1996
(51) Int. Cl.: C12N 15/51, C12Q 1/68, G01N 27/26

(54) **HUMAN HEPATITIS B VIRUS DNA**

(30) Priority: 02.11.1995 JP 285699/95
(71) Applicant: OTSUKA PHARMACEUTICAL CO., LTD., Chiyoda-ku Tokyo 101 (JP)
(72) Inventor: KINOSHITA, Moritoshi, Itano-gun, Tokushima 771-12 (JP); KATSURAGI, Kiyonori, Awa-gun, Tokushima 771-16 (JP)
(74) Representative: Kolb, Helga, Dr. Dipl.-Chem.
(86) International application number: JP9603108
(87) International publication number: WO9716551

(57) **Abstract**

A pair of PCR primers designed so that the regions 1701 to 1794 of a human hepatitis B virus are selectively amplified and represented by, for example, SEQ ID NOS: 1 and 2; and DNA fragments (standards) consisting of the regions 1701 to 1794 of a human hepatitis B virus and represented by, for example, SEQ ID NOS: 3 to 8. The use of the same makes it possible to conduct genotyping of variations in the Pre-C region of a human hepatitis B virus genome and assaying of each genotype simultaneously therewith.

## Description

### TECHNICAL FIELD

The present invention relates to a detecting method for human hepatitis B virus (HBV) and more particularly to a method for concurrent genotyping and assay of the precore (Pre-C) region of HBV and to DNA reagents for use in the method.

### BACKGROUND ART

Since 1976 when Greenberg and coworkers first reported the use of an interferon (IFN) in the treatment of type B chronic hepatitis [Greenberg, H. B., N. Engl., J. Med., 295, 517-522, 1975], many reports have appeared on the efficacy of IFN in HBe antigen-positive type B chronic active hepatitis and won more or less acceptance. Generally, type B chronic hepatitis runs a course characterized by an HBe antigen-positive stage in which HBV undergoes active replication, with blood HBV DNA and DNA polymerase activity levels being elevated, histologically a picture of active hepatitis is evident, and, in many cases, progression of hepatic lesions is noted and an ensuing stage in which HBe antigen disappears and HBe antibody appears instead (seroconversion occurs) so that replication of HBV subsides with remission of the hepatic lesions ensuing. Therefore, in chronic active hepatitis which is HBe antigen-, HBV DNA-, and DNA polymerase-positive, INFs are administered in hopes of encouraging clearance of HBe antigen and seroconversion in favor of HBe antibodies.

However, there exists type B chronic hepatitis which, despite being HBe antigen-negative or HBe antibody-positive, shows high blood HBV DNA and DNA polymerase levels, rapid progression, and poor prognosis. The DNA of the HBV detected in this hepatitis shows a mutation of G to A in the 83-position of the Pre-C (precore) region which is associated with secretion of HBe antigen into blood and, therefore, this HBV is considered to be a non-HBe antigen-producing virus [Carman W. J., et al., Lancet, ii, 588-591, 1989].

In the presence of such a Pre-C mutation, assayed values of HBe antibody and antigen may lead to the misdiagnosis that seroconversion is going on and, hence, remission of hepatic lesions will occur in due course. Therefore, in the IFN therapy of type B chronic hepatitis, detecting and quantitating mutations of the above-mentioned region of the DNA are considered to provide useful markers for planning out a future therapeutic schedule or predicting prognosis of the therapy.

The known procedure for detecting and assaying mutations in the Pre-C region of HBV is a competitive reaction assay by MSSA using a primer set having the same sequence as that of the 83-position mutation of the Pre-C region [Kinoshita, M., et al., CCA, 228, 83-90, 1994].

However, the above method has the following disadvantages.
(1) Because the method involves competitive reactions with 8 different standards from 10¹ to 10⁸ copies, the assay must be carried out 8 times for each one sample so that a time-consuming procedure is necessary in order to test a large number of samples.
(2) The assay is limited to the Pre-C 83-position mutant and the wild-type virus and other mutants cannot be simultaneously assayed.

On the other hand, polymerase chain reaction-single strand conformation polymorphism (PCR-SSCP) analysis is known as a mutation detecting method [e.g. Orita M., et al., Genomics 5, 874-879, 1989]. This method utilizes the change in electrophoretic mobility with conformational variation of the single-strand DNA. In this SSCP analysis, it is ideally preferable that the test DNA assume a unique conformation and, hence, gives a single band but it is known that actually a test DNA may assume several metastable structures giving several bands, thus imposing a restriction on the utility of the method [Analytical Chemistry, 43, 731-743, 1994].

No report is available on the qualitative analysis or identification of mutants of the HBV Pre-C region by means of PCR-SSCP analysis and, for that matter, a methodology for simultaneous detection and assay of such a mutation is not known.

### DISCLOSURE OF THE INVENTION

An object of the present invention is to provide DNA reagents and a method for genotyping mutants of HBV Pre-C region and the wild-type virus and simultaneously assaying the genotypes by a single reaction procedure.

For accomplishing the above object, the inventors made intensive investigations for identification of mutants of HBV Pre-C region by means of the PCR-SSCP analysis and discovered a specific region which includes all mutation sites of the HBV Pre-C region mutants to be determined and capable of assuming only one conformation specific to each of the mutant DNAs and the wild-type virus DNA and, hence, giving a single band each in SSCP analysis, with a sufficient difference in mobility to allow the DNAs to be differentiated in SSCP analysis. The present invention has been completed on the basis of the above findings.

The present invention provides a DNA fragment comprising the 1701-1794 region of HBV DNA.

In another aspect, the present invention provides the DNA fragment wherein the HBV is a wild-type virus or a mutant virus having at least one mutation in the Pre-C region as selected from the class consisting of substitution of A for G in 38-position, substitution of C for T in 42-position, substitution of C for T in 80-position, substitution of A for G in 83-position, and substitution of A for G in 86-position.

The present invention further provides a DNA fragment having a sequence selected from the class consisting of SEQ ID NO:3, NO:4, NO:5, NO:6, NO:7, and NO:8.

In a further aspect, the present invention provides a PCR primer pair designed to selectively amplify the 1701-1794 region of HBV DNA, preferably a primer pair of SEQ ID NO:1 and NO:2.

The present invention further provides a HBV test method which comprises using the primer pair and, as a standard, the DNA fragment.

The above-mentioned mutants of the Pre-C region involving substitution of A for G in 38-position, substitution of C for T in 42-position, substitution of C for T in 80-position, and substitution of A for G in 86-position, respectively, are considered to be novel mutants provided by the present invention.

The abbreviations used for amino acids, peptides, nucleotide sequences, nucleic acids, restriction enzymes, etc. in this specification are in accordance with the nomenclature recommended by IUPUC and IUPUC-IUB and the Guideline for Drafting of Specifications Including Nucleotide Sequences or Amino Acid Sequences (November 1994, the Examination Standard Group, Coordination Section of the Patent Office of Japan).

Further, the nucleotide sequence number of HBV as used in this specification is in accordance with that of the known nucleotide sequence [Gene, 30, 227-232, 1984].

The primer pair according to the present invention is characterized in that it has been designed to selectively amplify the 1701-1794 region of the HBV DNA. Design of the primer can be made by any routine procedure only if the region can be amplified.

Preferred examples of the primer so designed are the primer pair of SEQ ID NO:1 and NO:2 as described in the examples hereinafter.

The primer (oligo-DNA) of SEQ ID NO:1 according to the invention has a nucleotide sequence of the plus chain of HBV DNA Pre-C region which enables amplification of the above-mentioned specific region.

The primer (oligo-DNA) of SEQ ID NO:2 according to the invention has a sequence of the minus chain of the same region.

Those two primers can be successfully used as the primer pair of the invention and can be synthesized chemically, for example by the phosphoamidite method. Not only this chemical synthesis but also the various procedures used in the present invention such as enzymatic treatments for DNA cleavage, deletion, addition or splicing, and DNA isolation, purification, transcription, selection, and like procedures, can all be performed in the conventional manner [Bunshi-Idengaku Jikkenho (Experimental Protocols in Molecular Genetics), Kyoritsu Shuppan, 1983; PCR Technology, Takara Shuzo, 1990].

For example, DNA isolation and purification can be carried out by agarose gel electrophoresis and DNA determination can be carried out, for example, by the dideoxy method [Sanger, F., Nicklen, S. and Coulson, A. R., Proc. Natl. Acad. Sci., U.S.A., 74, 5463-5467, 1977] or the Maxam-Gilbert method [Maxam, A. M. and Gilbert, W., Method in Enzymology, 65, 499-560, 1980]. The sequencing of the DNA can be easily carried out by using a commercial sequence kit as well. Those basic procedures are used in the various reported studies referred to in this specification as well and reference can be made to those literature and the examples given hereinafter.

The DNA fragment consisting in the 1701-1794 region of HBV DNA as provided by the present invention is a specific region including all the mutation sites of the HBV Pre-C mutants to be determined by PCR-SSCP analysis, which may assume only one conformation specific to each of the mutant and the wild-type virus DNAs and hence give a single mobility band each in SSCP analysis, with a sufficient difference in mobility to allow the DNAs to be differentiated in SSCP analysis.

The DNA fragment according to the invention is the DNA fragment of the region of a wild-type HBV or a DNA fragment of the region of a mutant virus having at least one mutation selected from the class consisting of the mutations of Pre-C region involving substitution of A for G in 38-position, substitution of C for T in 42-position, substitution of C for T in 80-position, substitution of A for G in 83-position, and substitution of A for G in 86-position. More preferably, DNA fragments having the sequences of SEQ ID NO:3, NO:4, NO:5, NO:6, NO:7, and NO:8 can be mentioned.

The DNA fragment of SEQ ID NO:3 according to the invention is characterized in that, in the sequence of HBV Pre-C region, G in 83-position has mutated to A and T in 42-position has mutated to C.

The DNA fragment of SEQ ID NO:4 according to the invention is characterized in that, in the sequence of Pre-C region, G in 86-position has mutated to A.

The DNA fragment of SEQ ID NO:5 according to the invention is characterized in that, in the sequence of Pre-C region, G in 83-position has mutated to A.

The DNA fragment of SEQ ID NO:6 according to the invention is characterized in that it has the wild-type virus sequence of the Pre-C region.

The DNA fragment of SEQ ID NO:7 according to the invention is characterized in that, in the sequence of Pre-C region, G in 83-position has mutated to A and T in 80-position has mutated to C.

The DNA fragment of SEQ ID NO:8 according to the invention is characterized in that, in the sequence of Pre-C region, G in 83-position has mutated to A and C in 83-position has mutated to T.

All of those DNA fragments can be used with advantage as the standards for the genotyping of the Pre-C region and assay of the particular virus genotype in the HBV test by PCR-SSCP analysis.

The test procedure is now described in detail.

This test method utilizing PCR-SSCP analysis can be carried out in accordance with conventional protocols or with reference to the protocols. For example, reference can be made to the following literature for information on the PCR for amplifying a specific region of a DNA [Saiki, R. K., Scharf, S., Faloona, F. A., Mullis, K. B., Horn, G. T., Erlich, H. A. and Arnheim, N., Science, 230, 1350-1354, 1985, etc.]. As to SSCP analysis, the following literature can be referred to as a reference [Makino, R., et al., Cold Spring Harbor Lab., 2, 10-13, 1992, etc.].

Directed to analysis of a single-stranded DNA by PCR-SSCP, this technology is a method for assaying HBV in test samples which features the following steps.
(1) A step of amplifying the HBV DNA in a test sample by PCR using the primer pair of the invention.
(2) A step of analyzing the amplification product obtained in step (1) by the SSCP method.

In this assay method, the DNA fragment of the invention as prepared in known graded concentrations can be used as standards. By comparing the mobility and peak area of each standard as measured by SSCP analysis with the mobility and peak area of the test sample as the amplification product of step (1), detection of any mutation in the Pre-C region of HBV DNA (genotyping) and the assay of the particular virus can be simultaneously accomplished.

In this connection, the test sample containing the HBV DNA to be determined is not particularly restricted only if the DNA is contained, thus including blood, serum, sweat, urine, isolated tissues, and other biological materials. The HBV DNA can be extracted from those materials, purified, and prepared by the routine procedures.

In accordance with the method of the present invention, the particular region of positions 1701-1794 of the HBV DNA in the test sample is specifically amplified by PCR using the primer pair of the invention. The amplified regions contain HBV DNA Pre-C nucleotide positions 38, 42, 80, 83, and 86 and contain mutations at those positions. Regardless of the presence or the absence of mutations at said positions, the amplification products (DNA fragments) respectively assume one conformation specific to each DNA, with the result that, in the subsequent SSCP analysis in step (2); they respectively give a single band with a sufficient difference in mobility to permit the DNAs to be differentiated.

Therefore, by comparing the predetermined electrophoretic mobilities of such standard DNA fragments with the mobility of the test sample as amplified in step (1), the detection of mutations (genotyping) in the Pre-C region of HBV DNA can be practiced expediently and with good sensitivity.

Furthermore, when the standards are used in known graded concentrations, the virus titer in the test sample can be simultaneously assayed by comparing the peak areas of the standards with the peak area of the amplification product of the sample.

Therefore, the present invention provides an expedient method for concurrent detection of HBV DNA Pre-C region mutation (genotyping) and assay of the mutant.

An embodyment of the test method according to the present invention is now described in detail. The primer pair, standards, PCR-SSCP analysis, detection means, etc. for use in the method can be liberally modified by one skilled in the art based on the disclosure in this specification and, of course, such modifications or changes also fall within the scope of the present invention.

In accordance with this embodyment of the method of the invention, the virus DNA is extracted from the serum of a patient with type B hepatitis by the conventional procedure, for example by alkali or acid treatment, in the first place. Then, the primer pair, i.e. the primer of SEQ ID NO:2 and the fluorescence-labeled primer of SEQ ID NO:1, and a heat-resistant DNA polymerase are permitted to act upon the DNA solution obtained as above and the DNA fragment thus labeled is amplified.

On the other hand, the DNA fragments of SEQ ID NO:3, NO:4, NO:5, NO:6, NO:7, and NO:8 are respectively ligated to plasmid vectors and used to transfect Escherichia coli to provide transformants. After large-scale culture and purification, the purified recombinant plasmids are respectively used to prepare standards of, for example, 10³ copies, 10⁴ copies, 10⁵ copies, 10⁶ copies, 10⁷ copies, and 10⁸ copies. Then, the primer pair, i.e. the primer of SEQ ID NO:2 and the fluorescence-labeled primer of SEQ ID NO:1, and the heat-resistant DNA polymerase are permitted to act upon the respective standards and the labeled DNA fragments are amplified.

The amplified DNA-solutions are heated at about 95°C for about 5 minutes, cooled immediately in ice, and fed to an automatic sequencer for SSCP analysis, whereby fluorescent peaks are detected. The electrophoresis in this SSCP analysis is preferably carried out at about 30±1°C.

Thus, the patient serum DNA peak (mobility) is compared with the peaks (mobility) of the standards. The genotyping of the patient HBV can thus be made by identifying the peak in agreement with a standard peak from migration time. Moreover, by calculating the peak areas of the respective standards and constructing a standard curve, the HBV DNA can be quantitated (assayed) from the calculated peak area of the patient DNA.

In accordance with the present invention, there is provided a primer pair which is characterized in that the Pre-C region of HBV DNA can be amplified by PCR and the resultant amplification product be detected as a single peak by SSCP analysis. The present invention further provides a DNA fragment of the same region as the amplification product which is useful as a standard for the wild-type virus and a mutant of the HBV. By utilizing said primer pair in combination with such standards, the genotyping of the Pre-C region of HBV DNA and the assay of the virus DNA can be simultaneously carried out by one round of PCR.

### BRIEF DESCRIPTION OF THE DRAWINGS

Fig. 1 is a graph showing the results of PCR-SSCP analysis of the Pre-C region of human hepatitis B virus DNA in accordance with Example 1.

Fig. 2 is a graph showing the standard curve for the PCR-SSCP analysis of the Pre-C region of human hepatitis B virus DNA in accordance with Example 1.

### BEST MODE FOR CARRYING OUT THE INVENTION

The following examples illustrate the present invention in further detail and should by no means be construed as defining the scope of the invention.

### Example 1

### 1-1. Preparation of primers

The two primers used in this example were HBV E-1 and HBV E-2. HBV E-1 is an oligo DNA chemically synthesized according to SEQ ID NO:1 and HBV E-2 is an oligo DNA chemically synthesized according to SEQ ID NO: 2.

Both of said primers were synthesized by using a DNA synthesizer (Pharmacia LKB Gene Assembler Plus).

### 1-2. Preparation of standards

The DNA fragments used as standards were prepared as follows. Thus, mutants were identified by sequencing the DNA in the sera from patients with chronic type B hepatitis by the procedure described below and the DNA fragments corresponding to SEQ ID NO:3, NO:4, NO: 5, NO: 6, NO: 7, and NO: 8, respectively, were amplified by PCR. The amplified standard DNA fragments had sequences of SEQ ID NO:3, NO:4, NO:5, NO:6, NO:7, and NO:8, respectively.

Each of the above DNA fragments was inserted into the plasmid vector (PT7 Blue T-Vector, Takara Shuzo) and used to transfect Escherichia coli. From the transformant cells cultured on a large scale, the recombinant plasmid was purified. Using the recombinant plasmids, the respective standard solutions were prepared using sterilized water containing 200 µg/ml of denatured salmon DNA at the concentrations of 10³ copies/µl for the DNA fragment of SEQ ID NO:3, 10⁴ copies for the DNA fragment of SEQ ID NO:4, 10⁵ copies/µl for the DNA fragment of SEQ ID NO:5, 10⁶ copies/µl for the DNA fragment of SEQ ID NO:6, 10⁷ copies/µl for the DNA fragment of SEQ ID NO:7, and 10⁸ copies/µl of the DNA fragment of SEQ ID NO:8.

### 1-3. Extraction of the hepatitis B virus DNA

From the blood of a patient with chronic type B hepatitis, the serum was separated and a 10 µl portion of the serum was taken in a tube. To this tube, 10 µl of 0.2N-NaOH was added, and the mixture was stirred vigorously for 5 minutes and then allowed to stand at 37°C for 20 minutes to extract the DNA. This DNA solution was neutralized by adding 10 µl of 0.2N-HCl for use in PCR.

### 1-4. PCR

One µl each of the standard solutions prepared in 1-2 and 5 µl of the patient serum DNA solution prepared in 1-3 were respectively taken in fresh tubes. To each tube, 5 µl each of an FITC-labeled (Pharmacia) HBV E-1 solution (10 µM FITC-labeled HBV E-1 in sterile distilled water) and an HBV E-2 solution (10 µM HBV E-2 in sterile distilled water) were added, followed by addition of 30 µl of the PCR enzyme solution. After the total volume was adjusted to 50 µl with sterile distilled water and following addition of mineral oil, an amplification reaction was carried out in a PCR machine (Stratagene) in 30 cycles of 94°C for 1 minute, 58°C for 1.5 minutes, and 72°C for 1.5 minutes.

### 1-5. Genotyping and assay by SSCP analysis

One µl of the PCR product solution obtained in 1-4 was transferred to a fresh tube and 10 µl of stop-denature solution (80% deionized formamide, 0.01% blue dextran) was added. The mixture was heated at 95°C for 3 minutes, cooled immediately in ice, and an SSCP analysis was carried out by 5% glycerol-7% polyacrylamide gel electrophoresis using an ALF automatic sequencer (Pharmacia).

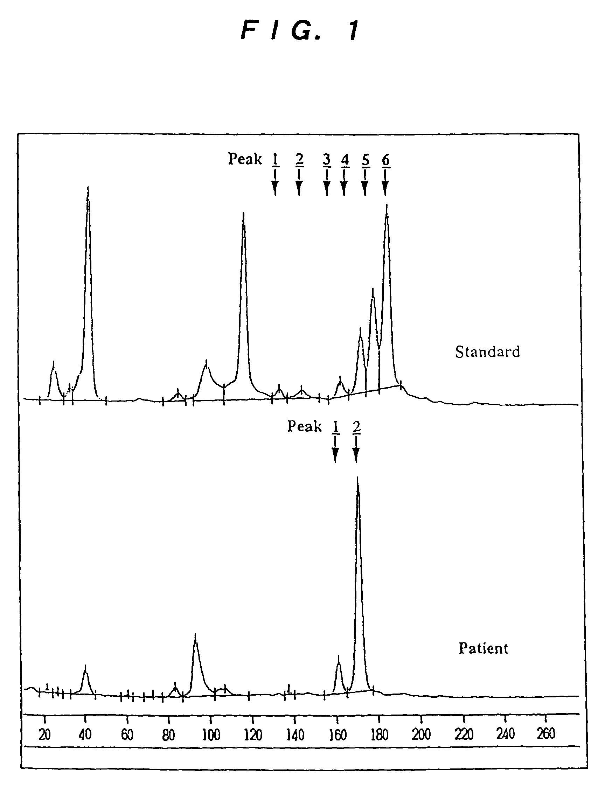

The results are shown in Fig. 1. In the diagram, the ordinate represents fluorescence intensity and the abscissa represents mobility. The top row (Standard) shows the result obtained by running a mixture of standards. The six mutant-derived peaks 1, 2, 3, 4, 5, and 6, indicated by arrowmarks, correspond to the DNA fragments of SEQ ID NO:3, NO:4, NO:5, NO:6, NO:7, and NO:8, respectively.

It can be seen from the diagram that the peaks of the respective standards appear as completely segregated singlets and that the peak areas correspond to their concentrations.

On the other hand, the bottom row (Patient) shows the result for the PCR product derived from the patient serum. This patient gave two peaks 1 and 2 as indicated by arrowmarks. Comparison of the mobilities of the two peaks with the above mobilities of the standards revealed that the patient peak 1 agreed with the standard peak 3 (SEQ ID NO:5), indicating that it represents a genotype such that G in 83-position of the Pre-C region had mutated to A. The patient peak 2 agreed with standard peak 4 (SEQ ID NO:6) and was, therefore, found to be the wild-type virus.

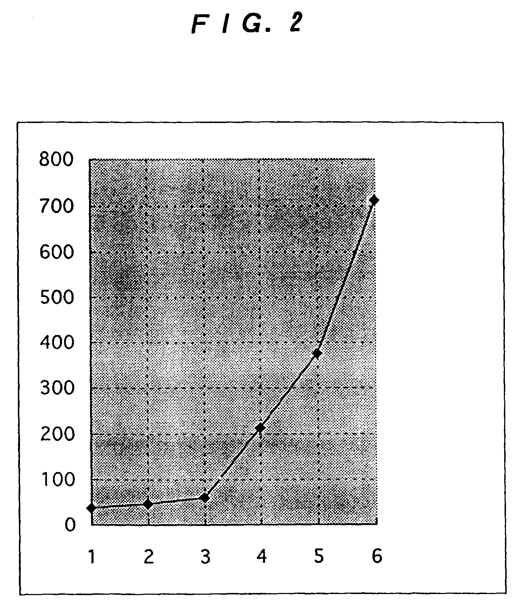

In addition, the peak areas of the standards were respectively calculated (Fragment Manager, Pharmacia) from Fig. 1. As a result, the peak area was 37 for peak 1, 46 for peak 2, 60 for peak 3, 212 for peak 4, 375 for peak 5, and 713 for peak 6. The standard curve constructed using the above data are shown in Fig. 2 (ordinate: peak area, abscissa: peak no.).

The peak areas of the patient peaks 1 and 2 were similarly calculated (peak 1: 220, peak 2: 1372) and the respective populations were determined from the above standard curve. As a result, the population of patient peak 1 was 3x10⁶ copies and that of patient peak 2 was 1x10¹² copies.

Thus, the patient serum used in this test contained 3x10⁶ copies of the mutant HBV with a mutation of G to A in the 83-position of the Pre-C region of HBV and 1x10¹² copies of the wild-type HBV.

### INDUSTRIAL APPLICABILITY

The present invention provides a method for carrying out the genotyping of mutations in the Pre-C region of human hepatitis B virus (HBV) genome and the assay of the respective genotypes in one operation and the associated primers and standard DNA fragments with which an accurate HBV test can be performed.

SEQUENCE LISTING
SEQ ID NO:1:
LENGTH: 22 base pairs
STRANDEDNESS: single
TOPOLOGY: linear
MOLECULE TYPE: synthetic DNA
ORIGINAL SOURCE: human hepatitis B virus DNA
LOCATION: 1701..1722
SEQUENCE DESCRIPTION:
ACCTCTGCCT AATCATCTCA TG 22
SEQ ID NO:2:
LENGTH: 20 base pairs
STRANDEDNESS: single
TOPOLOGY: linear
MOLECULE TYPE: synthetic DNA
ORIGINAL SOURCE: human hepatitis B virus DNA
LOCATION: 1775..1794
SEQUENCE DESCRIPTION:
TTATACGGGT CAATGTCCAT 20
SEQ ID NO:3:
LENGTH: 94 base pairs
STRANDEDNESS: double
TOPOLOGY: linear
MOLECULE TYPE: PCR product
ORIGINAL SOURCE: human hepatitis B virus DNA

FEATURE:

NAME/KEY: mutation

LOCATION: 1701..1794

SEQUENCE DESCRIPTION:

ACCTCTGCCT AATCATCTCA TGTTCATGCC CTACTGTTCA AGCCTCCAAG CTGTGCCTTG 60

GGTGGCTTTA GGGCATGGAC ATTGACCCGT ATAA 94

SEQ ID NO:4:

LENGTH: 94 base pairs

STRANDEDNESS: double

TOPOLOGY: linear

MOLECULE TYPE: PCR product

ORIGINAL SOURCE: human hepatitis B virus DNA

FEATURE:

NAME/KEY: mutation

LOCATION: 1701..1794

SEQUENCE DESCRIPTION:

ACCTCTGCCT AATCATCTCA TGTTCATGTC CTACTGTTCA AGCCTCCAAG CTGTGCCTTG 60

GGTGGCTTTG GGACATGGAC ATTGACCCGT ATAA 94

SEQ ID NO:5:

LENGTH: 94 base pairs

STRANDEDNESS: double

TOPOLOGY: linear

MOLECULE TYPE: PCR product

ORIGINAL SOURCE: human hepatitis B virus DNA

FEATURE:

NAME/KEY: mutation
LOCATION: 1701..1794
SEQUENCE DESCRIPTION:
ACCTCTGCCT AATCATCTCA TGTTCATGTC CTACTGTTCA AGCCTCCAAG CTGTGCCTTG 60
GGTGGCTTTA GGGCATGGAC ATTGACCCGT ATAA 94
SEQ ID NO:6:
LENGTH: 94 base pairs
STRANDEDNESS: double
TOPOLOGY: linear
MOLECULE TYPE: PCR product
ORIGINAL SOURCE: human hepatitis B virus DNA
FEATURE:
LOCATION: 1701..1794
SEQUENCE DESCRIPTION:
ACCTCTGCCT AATCATCTCA TGTTCATGTC CTACTGTTCA AGCCTCCAAG CTGTGCCTTG 60
GGTGGCTTTG GGGCATGGAC ATTGACCCGT ATAA 94
SEQ ID NO:7:
LENGTH: 94 base pairs
STRANDEDNESS: double
TOPOLOGY: linear
MOLECULE TYPE: PCR product
ORIGINAL SOURCE: human hepatitis B virus DNA
FEATURE:
NAME/KEY: mutation
LOCATION: 1701..1794

SEQUENCE DESCRIPTION:

ACCTCTGCCT AATCATCTCA TGTTCATGTC CTACTGTTCA AGCCTCCAAG CTGTGCCTTG 60

GGTGGCCTTA GGGCATGGAC ATTGACCCGT ATAA 94

SEQ ID NO:8:

LENGTH: 94 base pairs

STRANDEDNESS: double

TOPOLOGY: linear

MOLECULE TYPE: PCR product

ORIGINAL SOURCE: human hepatitis B virus DNA

FEATURE:

NAME/KEY: mutation

LOCATION: 1701..1794

SEQUENCE DESCRIPTION:

ACCTCTGCCT AATCATCTCA TGTTTATGTC CTACTGTTCA AGCCTCCAAG CTGTGCCTTG 60

GGTGGCCTTA GGGCATGGAC ATTGACCCGT ATAA 94

## Claims

1. A PCR primer pair comprising being designed to selectively amplify the 1701-1794 region of human hepatitis B virus DNA.

2. The primer pair according to Claim 1 which is represented by SEQ ID NO:1 and NO:2.

3. A DNA fragment comprising the 1701-1794 region of human hepatitis B virus DNA.

4. The DNA fragment according to Claim 3 wherein the human hepatitis B virus is a wild-type virus or a mutant virus having at least one mutation in the precore region as selected from the class consisting of substitution of A for G in 38 position, substitution of C for T in 42 position, substitution of C for T in 80 position, substitution of A for G in 83 position, and substitution of A for G in 86 position.

5. The DNA fragment according to Claim 3 which has a sequence selected from the class consisting of the sequences represented by SEQ ID NO:3, NO:4, NO:5, NO:6, NO:7, and NO:8.


SEQUENCE LISTING

(1) GENERAL INFORMATION:

(i) APPLICANT: Ostuka Pharmaceutical Co., Ltd.

(ii) TITLE OF INVENTION: Human hepatitis B virus DNA

(iii) NUMBER OF SEQUENCES: 8

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vii) PRIOR APPLICATION DATA:
(A) APPLICATION NUMBER: PCT/JP96/03108
(B) FILING DATE: 24-OCT-1996

(2) INFORMATION FOR SEQ ID NO:1:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO:1:

ACCTCTGCCT AATCATCTCA TG
22

(2) INFORMATION FOR SEQ ID NO:2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid

(xi) SEQUENCE DESCRIPTION: SEQ ID NO:2:

TTATACGGGT CAATGTCCAT
20

(2) INFORMATION FOR SEQ ID NO:3:

(i) SEQUENCE CHARACTERISTICS:
(A) LENGTH: 94 base pairs
(B) TYPE: nucleic acid
(C) STRANDEDNESS: double
(D) TOPOLOGY: linear

(ii) MOLECULE TYPE: genomic DNA

(vi) ORIGINAL SOURCE:
(A) ORGANISM: human hepatitis B virus DNA

(xi) SEQUENCE DESCRIPTION: SEQ ID NO:3:

ACCTCTGCCT AATCATCTCA TGTTCATGCC CTACTGTTCA AGCCTCCAAG CTGTGCCTTG
60
GGTGGCTTTA GGGCATGGAC ATTGACCCGT ATAA
94

(2) INFORMATION FOR SEQ ID NO:4:

(i) SEQUENCE CHARACTERISTICS:
(A) LENGTH: 94 base pairs
(B) TYPE: nucleic acid
(C) STRANDEDNESS: double
(D) TOPOLOGY: linear

(ii) MOLECULE TYPE: genomic DNA

(vi) ORIGINAL SOURCE:
(A) ORGANISM: human hepatitis B virus DNA

(xi) SEQUENCE DESCRIPTION: SEQ ID NO:4:

ACCTCTGCCT AATCATCTCA TGTTCATGTC CTACTGTTCA AGCCTCCAAG CTGTGCCTTG
60
GGTGGCTTTG GGACATGGAC ATTGACCCGT ATAA
94

(2) INFORMATION FOR SEQ ID NO:5:

(i) SEQUENCE CHARACTERISTICS:
(A) LENGTH: 94 base pairs
(B) TYPE: nucleic acid
(C) STRANDEDNESS: double
(D) TOPOLOGY: linear

(ii) MOLECULE TYPE: genomic DNA

(vi) ORIGINAL SOURCE:
(A) ORGANISM: human hepatitis B virus DNA

(xi) SEQUENCE DESCRIPTION: SEQ ID NO:5:

ACCTCTGCCT AATCATCTCA TGTTCATGTC CTACTGTTCA AGCCTCCAAG CTGTGCCTTG
60
GGTGGCTTTA GGGCATGGAC ATTGACCCGT ATAA
94

(2) INFORMATION FOR SEQ ID NO:6:

(i) SEQUENCE CHARACTERISTICS:
(A) LENGTH: 94 base pairs
(B) TYPE: nucleic acid
(C) STRANDEDNESS: double
(D) TOPOLOGY: linear

(ii) MOLECULE TYPE: genomic DNA

(vi) ORIGINAL SOURCE:
(A) ORGANISM: human hepatitis B virus DNA

(xi) SEQUENCE DESCRIPTION: SEQ ID NO:6:

ACCTCTGCCT AATCATCTCA TGTTCATGTC CTACTGTTCA AGCCTCCAAG CTGTGCCTTG
60
GGTGGCTTTG GGGCATGGAC ATTGACCCGT ATAA
94

(2) INFORMATION FOR SEQ ID NO:7:

(i) SEQUENCE CHARACTERISTICS:
(A) LENGTH: 94 base pairs
(B) TYPE: nucleic acid
(C) STRANDEDNESS: double
(D) TOPOLOGY: linear

(ii) MOLECULE TYPE: genomic DNA

(vi) ORIGINAL SOURCE:
(A) ORGANISM: human hepatitis B virus DNA

(xi) SEQUENCE DESCRIPTION: SEQ ID NO:7:

ACCTCTGCCT AATCATCTCA TGTTCATGTC CTACTGTTCA AGCCTCCAAG CTGTGCCTTG
60
GGTGGCCTTA GGGCATGGAC ATTGACCCGT ATAA

(2) INFORMATION FOR SEQ ID NO:8:

(i) SEQUENCE CHARACTERISTICS:
(A) LENGTH: 94 base pairs
(B) TYPE: nucleic acid
(C) STRANDEDNESS: double
(D) TOPOLOGY: linear

(ii) MOLECULE TYPE: genomic DNA

(vi) ORIGINAL SOURCE:
(A) ORGANISM: human hepatitis B virus DNA

(xi) SEQUENCE DESCRIPTION: SEQ ID NO:8:

ACCTCTGCCT AATCATCTCA TGTTTATGTC CTACTGTTCA AGCCTCCAAG CTGTGCCTTG
60
GGTGGCCTTA GGGCATGGAC ATTGACCCGT ATAA
94